Europäisches Patentamt

**European Patent Office**

**Office européen des brevets**

(11) Publication number: **0 325 347**
**A1**

## (12) EUROPEAN PATENT APPLICATION

(21) Application number: **89300116.4**

(22) Date of filing: **06.01.89**

(51) Int. Cl.⁴: **A61F 2/38**

(30) Priority: **21.01.88 GB 8801278**

(43) Date of publication of application:
**26.07.89 Bulletin 89/30**

(84) Designated Contracting States:
**DE FR**

(71) Applicant: **J.E. HANGER & COMPANY LIMITED**
**Roehampton Lane**
**Roehampton London SW15 5PL(GB)**

(72) Inventor: **Cooper, John Edwin**
**St. Gallen The Ballands North**
**Leatherhead Surrey(GB)**

(74) Representative: **Moore, John Hamilton et al**
**BTR Group Patent & Trade Mark Service P.O.**
**Box 504**
**Erdington Birmingham B24 9QH(GB)**

(54) **Knee prosthesis.**

(57) A knee prosthesis for an artificial leg which comprises a top hinge member (1) and a lower hinge member (2) interconnected by link means (3),the top hinge member incorporating a brake member (6) with a convex face (27) which under download in or adjacent to an unflexed state of the knee acts against a seat (7) on the lower hinge member (2) to provide frictional resistance to flexing of the knee. Resilient biasing means (10) may be provided on the lower hinge member (2) to disengage the brake member (6) from its seat (7) in the absence of a download.

Fig.1.

EP 0 325 347 A1

## KNEE PROSTHESIS

This invention relates to a knee prosthesis, and more particularly to such a prosthesis having provision for stance phase control so that at small angles of knee flexion angular movement of thigh and shin parts of the leg are resisted when the weight of the patient is on the leg.

It is an object of the invention to provide a knee of the above kind that is not of unduly complex construction and that has an extended service life.

Accordingly the invention provides a knee prosthesis for an artificial leg comprising a top hinge member and a lower hinge member, the lower hinge member having a transverse central plane, said top and lower hinge members being interconnected by link means hinged to the top hinge member at a knee pivot located to the posterior side of the said transverse central plane and hinged to the lower hinge member at a stance control pivot to the anterior side of the transverse central plane, the top hinge member having a brake member having a convex face that under download of the top hinge member in or adjacent to an unflexed state of the knee acts against a seat on the lower member to provide a frictional resistance to angular movement of the top and lower hinge members.

The top hinge member will have a transverse central plane defined by attachment means for upper parts of the leg that in an unflexed state of the leg coincides with the transverse central plane of the lower hinge member and to avoid instability is not offset in an anterior or a posterior direction. In order to obtain a favourable load distribution in a download situation combined with good frictional resistance, a plane through the knee pivot axis and the stance control pivot may make an included angle of about 45 degrees to the transverse central plane of the lower hinge member, when the brake member is engaged with the seat but other angles such as 30° or 82° are possible alternatives. The contact line between the convex face of the brake member and the seat of the lower hinge member is advantageously located a short distance to the anterior of the transverse central plane of the lower hinge member - if it is positioned to the posterior side of that plane the available frictional resistance to on-load flexion or extension is reduced. Furthermore, the included angle between a first plane containing the knee pivot axis and the line of contact between the brake member and the seat and a second plane containing the knee and stance control pivot axes may be about 7 degrees, again for optimum force distribution. In particular the abutting steel surfaces that may be used for the brake member and seat have a relatively low coefficient of friction, and hence a high force normal to these surfaces is required for development of a tangential force necessary to overcome the applied moments.

A stance control buffer on the lower hinge member is preferably provided, against which a portion of the link means abuts, said buffer reducing noise while the patient is walking. The link means preferably comprises a pair of links to opposed sides of the top and bottom hinge members, cross-connector means attached to or integral with each link establishing a rigid mechanical connection between the links so as to resist loads resulting from side forces on the leg.

Although the stance control is operative without a means for biasing it to an inactive state, it is preferred to provide a positive bias into that state to prevent the leg locking in an unwanted attitude during walking, and preferably the amount of download required to make the device operative is adjustable to take account of the weight and activity level of the patient. Accordingly, means on the lower hinge member is preferably provided that is connected to the link means to bias it in a direction such as to disengage the brake member from the seat in the absence of a download.

Embodiments of the invention will now be described, by way of example only, with reference to the accompanying diagrammatic drawings in which :-

Figure 1 is a side view of an artificial knee with provision for stance control;

Figure 2 is a rear elevation of an alternative knee prosthesis in accordance with the invention;

Figure 3 is a section on the line III-III of Figure 2;

Figure 4 is a rear elevation of a further alternative knee prosthesis in accordance with the invention, and

Figure 5 is a section on the line V - V of Figure 4.

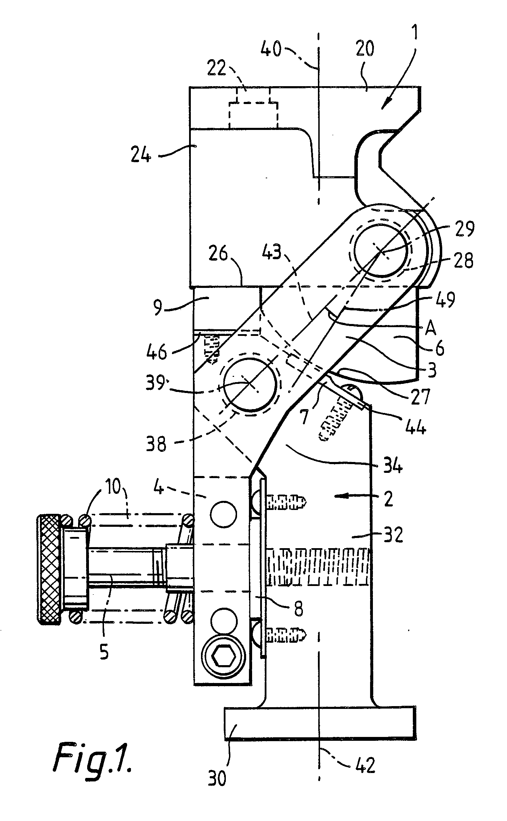

In Figure 1, an artificial knee comprises a top hinge member 1 formed with a top plate 20 having four fixing holes 22 (only one of which is shown) for attachment to upper parts of the leg, and a body portion 24 that is smaller when viewed in front profile than the profile viewed from the rear. The body portion 24 has a generally horizontal lower face 26 for reacting against an extension buffer 9. A knee pivot hole in the body 24 at a posterior location houses a pivot bush 28, and a brake member in the form of a cam block 6 having an arcuate convex lower face 27 concentric with the pivot bush 26 is attached by bolts or other suitable means to the lower face of the body 24 between the knee pivot bush 28 and the extension buffer 9. The cam block 6 is of hardened steel.

A lower hinge member 2 of the knee is of a forwardly facing angular shape when viewed in side profile and has a lower platform 30 formed with a pattern of four fixing holes (not shown) for attachment to shin parts of the leg, a post 32 extending upwardly from the platform, and a forwardly cranked nose 34. A second or stance control pivot hole is formed in the nose 34 and houses a pivot bush 38. The pattern of fixing holes in the plate 22 defines a transverse central plane 40 for the top hinge member 1 that in the unflexed position of the knee coincides with a transverse central plane 42 of the lower hinge member 2. The axis 29 of the knee pivot which is formed in the top hinge member 1 is about 10-12.5 mm behind the central plane 40, and the axis 39 of a stance control pivot which is formed in the nose 34 of the lower hinge 2 is forward of the central plane 42. The plane 43 joining pivot axes 29, 39 is directed at 45 degrees to the central planes 40, 42.

The top hinge 1 and the lower hinge 2 are joined by link means comprising connecting arms 3 of generally bell-crank side profile, with a rearwardly and upwardly facing limb carrying the pivot pin for the knee axis, and with the angulation area carrying a pin for the stance control pivot. A lower limb of each connecting arm 3 is located just forwardly of and in closely spaced generally parallel relationship to the post 32, and the lower limbs to either side of the knee are joined by a spacer block 4 to form a rigid Y-structure.

Because the knee pivot axis 29 is behind the centre lines 40, 42, download on the top hinge 1 rotates the connecting arms 3 clockwise as viewed in Figure 1, causing the cam block 6 to approach the post 32 and the lower limbs of the arms 3 to move away from the post 32. This movement is resisted by a compression coil spring 10 that acts between the spacing block 4 and the head of an adjusting screw 5, engaging a threaded hole in the post 32, that can be rotated in either direction to adjust the force exerted by spring 10 to suit patient requirements. When the weight is removed, the spring 10 returns the lower limbs of arms 3 towards the post 32 until block 4 abuts a stance control buffer 8′carried by the post 32.

The post 32 of the lower hinge member 2 has a forwardly and upwardly inclined rear portion 44 of its top face behind a horizontal portion 46 to which the extension buffer 9 is fixed. The included angle between the rear top face 44 and the central plane 42 is about 38 degrees, and the face 44 carries a hardened steel impact pad 7 providing a seat with which the cam block 6 makes generally line contact when weight is applied to the top hinge 1. The line of contact between the cam block 6 and the contact pad 7 is a small distance, typically 2-3 mm, to the anterior of the central plane 42, and remains at this position when weight is applied to a partially flexed knee. An angle A between a first plane 49 through the contact line and the knee pivot 29 and a second plane 43, the plane 43 being the plane containing the knee pivot axis 29 and the stance control pivot axis 39, is about 7 degrees. These values have been found to provide an appropriate load normal to the pad 7 to give rise to an appropriate resistance to flexion about the axis 29 without the forces involved being excessive and requiring over-stressed components. The angular extent of the curved face of the block 6 is sufficient to give stance control over about 25 degrees of knee flexion, and the knee illustrated is capable of flexion up to at least 130 degrees.

It will be appreciated that various modifications may be made to the embodiment described above without departing from the invention, the scope of which is defined in the appended claims. For example, other forms of biasing means to lift the cam block 6 from contact with the impact pad 7 may be provided. The lower extensions of the connecting arms 3 may be dispensed with and the resilient means may provide an upward load on the arms 3 at a position between the pivot axes 29, 39. It is preferred, however, that a rigid mechanical interconnection between the arms 3 to either side of the knee should be retained to resist twisting resulting from side loads on the leg.

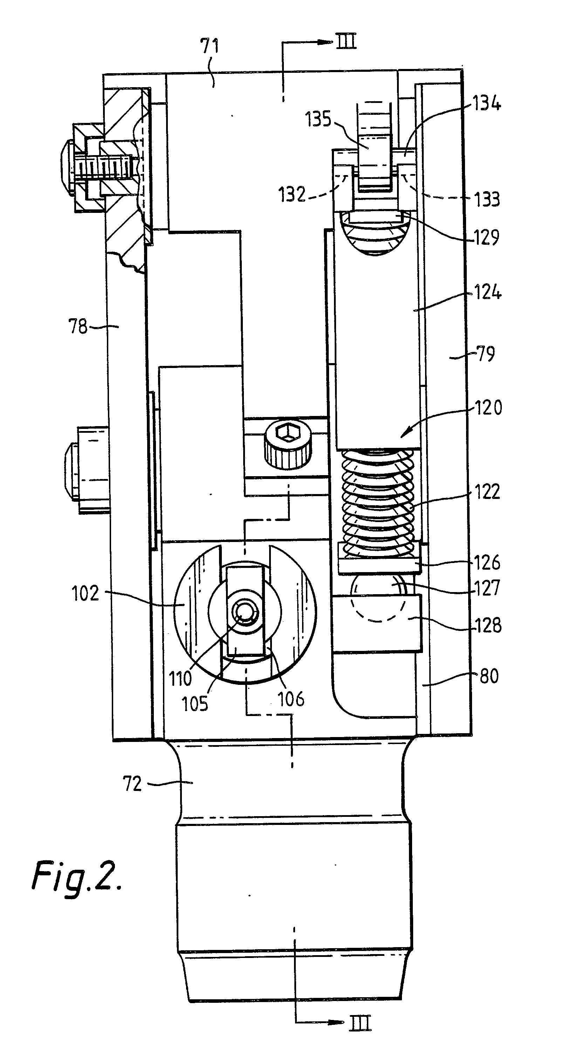

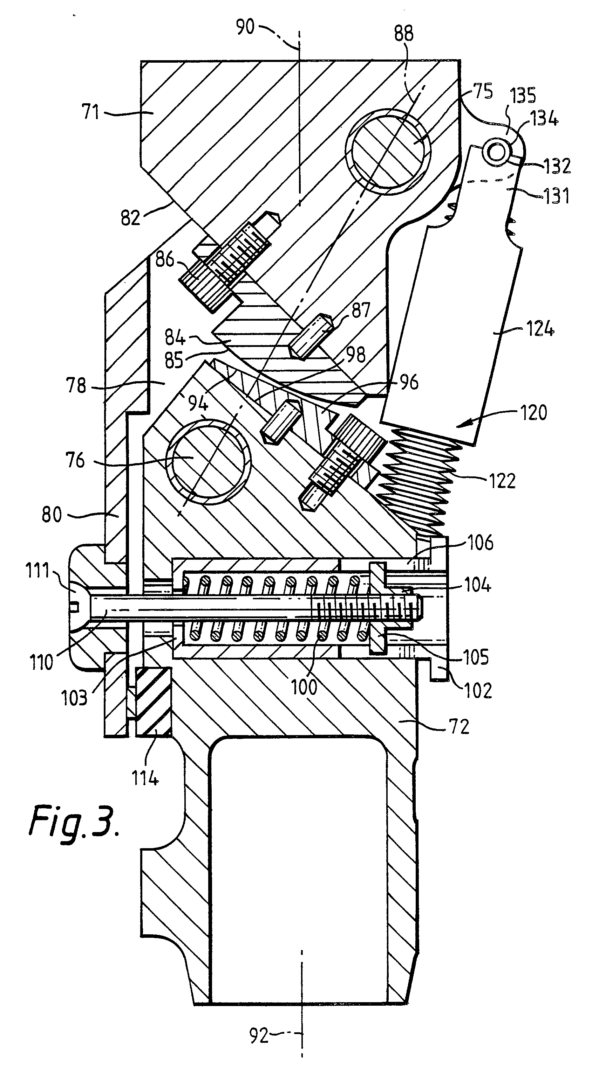

Figures 2 and 3 show an alternative knee prosthesis incorporating an upper hinge member 71 and a lower hinge member 72. A knee pivot 75 and a stance control pivot 76 are linked by connecting arms 78, 79 joined by a spacer block 80 as described in relation to the embodiment shown in Figure 1, the arrangement being generally similar to that of Figure 1 but differing in a number of respects which will be described.

The upper hinge member 71 incorporates an inclined surface 82 to which a brake member 84 having a convex surface 85 is secured by means of a screw 86 and a dowel pin 87. The arrangement is such that a plane 88 containing the pivot axes of the knee and stance pivots 75, 76 makes an angle of 30° to the transverse mid-plane 90 of the upper hinge member, which in the position illustrated coincides with the mid-plane 92 of the lower hinge member, and when a vertical download is applied to the upper hinge member the brake member moves towards a flat surface 94 of an impact pad 96 secured to an inclined surface 98 of the lower hinge member 72, the arms 78 and 79 tilting about the stance control pivot 76 and the plane 90 being slightly displaced relative to the plane 92.

Tilting of the arms 78, 79 is resisted by a compression spring 100 mounted in a housing 102 recessed into the lower hinge member 72 and having an abutment 103 for one end of the spring. The other end of the spring 100 abuts a nut 104 having wings 105 guided in slots 106 of the housing 102 to prevent rotation of the nut, and a pull rod 110 extends through the spring 100 for screw-threaded engagement with the nut 104 at one end and engagement with the spacer block 80 at its other end, the head 111 of the pull rod being slotted to permit the spring force to be adjusted by rotation of the pull rod by means of a screwdriver. A resilient pad 114 is provided to cushion impact between the spacer block 80 and the lower hinge member 72.

Extensor means in the form of a spring 120 for returning the knee to the unflexed condition comprises a stack of spring washers 122 threaded on a central extensible telescoping stem (not shown) and surrounded by an external telescoping sleeve 124. The central stem comprises a flange 126 to receive thrust from the stack of washers and transmit it via an integral ball 127 to a socket 128 on the lower hinge member, and a flange 129 which transmits thrust from the washers to the sleeve 124. The sleeve 124 comprises a forked portion 131 which has sockets 132, 133 engaging a pin 134 mounted in a lug 135 on the upper hinge member.

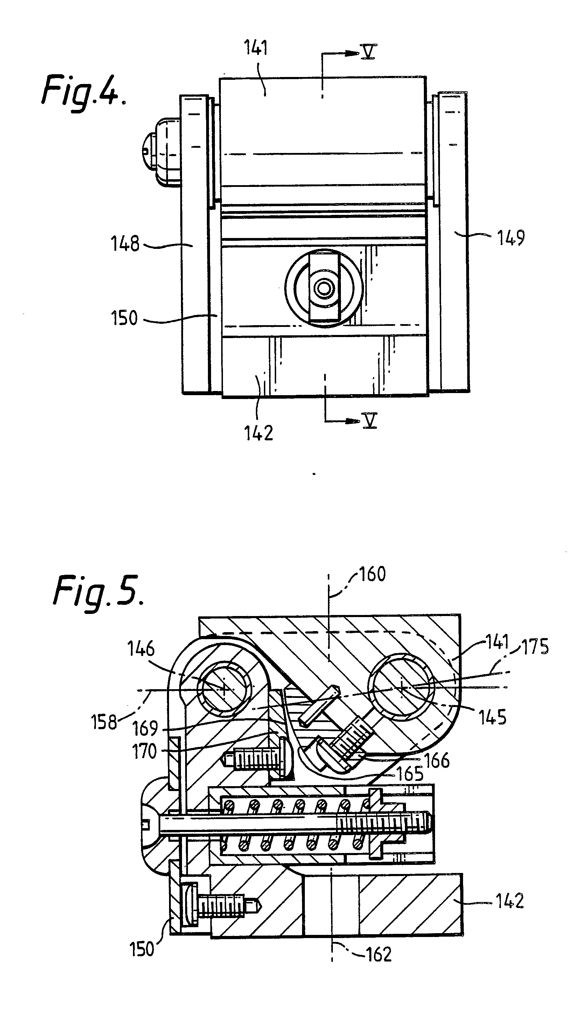

Figures 4 and 5 show a further alternative knee prosthesis in which an upper hinge member 141 and a lower hinge member 142 have a knee pivot axis 145 and a stance control pivot axis 146 linked by connecting arms 148, 149 which are joined by a spacer block 150. A line 158 indicates the plane containing the knee pivot and stance control pivot axes, and this plane is disposed substantially at right angles to the transverse mid-planes 160 and 162 of the upper and lower hinge members.

In the position shown in Figure 5 contact is established between a convex surface 165 of a brake member 166 secured to the upper hinge member and a plane surface 169 of an impact pad 170 fixed to the lower hinge member, as indicated by the line 175 representing the plane containing the contact line and the knee pivot axis 145.

The arrangement of Figures 5 and 6 provides a more rapid response to effect locking of the knee when it is in the unflexed state.

## Claims

1. A knee prosthesis for an artificial leg comprising a top hinge member (1;71;141) and a lower hinge member (2;72;142), the lower hinge member having a transverse central plane (42;92;162), characterised in that said top and lower hinge members are interconnected by link means (3;78,79;148,149) hinged to the top hinge member at a knee pivot (28;75;145) located to the posterior side of the said transverse central plane and hinged to the lower hinge member at a stance control pivot (38;76;146) located to the anterior side of the transverse central plane, the top hinge member having a brake member (6;84;166) having a convex face (27;85;165) that under download of the top hinge member in or adjacent to an unflexed state of the knee acts against a seat (7;94;169) on the lower hinge member to provide a frictional resistance to angular movement of the top and lower hinge members.

2. A knee prosthesis according to Claim 1, characterised in that the top hinge member has a transverse central plane (40;90;160) defined by attachment means for upper parts of the leg that in an unflexed state of the leg coincides with the transverse central plane (42;92;162) of the lower hinge member.

3. A knee prosthesis according to Claim 1 or 2, characterised in that a plane (43;88;158) containing the knee pivot axis and the stance control pivot axis makes an included angle of about 45° to the transverse central plane (42;92;162) of the lower hinge member.

4. A knee prosthesis according to Claim 1 or Claim 2 characterised in that a plane (43;88;158) containing the knee pivot axis and the stance control pivot axis makes an included angle of about 30° to the transverse central plane (42;92;162) of the lower hinge member.

5. A knee prosthesis according to Claim 1 or Claim 2 characterised in that a plane (43;88;258) containing the knee pivot axis and the stance control pivot axis makes an included angle of about 82° to the transverse central plane (42;92;162) of the lower hinge member.

6. A knee prosthesis according to any of Claims 3-5 characterised in that a contact line between the lower face (27;85;165) of the brake member (6;84;166) amd the seat (7;94;169) of the lower hinge member is located a short distance to the anterior of the transverse central plane (42;92;162) of the lower hinge member.

7. A knee prosthesis according to Claim 6, characterised in that the included angle (A) between a first plane (49;175) passing through the knee pivot axis and the line of contact between the brake member and the seat and a second plane (43;88;158) passing through the knee and stance control pivot axes is about 7°.

8. A knee prosthesis according to any preceding claim, characterised in that a stance control buffer (8;114) is provided on the lower hinge member against which portions (4;80) of the link means abut.

9. A knee prosthesis according to Claim 5, characterised in that the link means comprises a pair of links (3;78,79;148,149) to opposed sides of the top and bottom hinge members, cross-connector means (4;80;150) attached to or integral with each link establishing a rigid mechanical connection between the links.

10. A knee prosthesis according to any preceding claim, characterised in that resilient means (10;100) carried by the lower hinge member biases the link means (3;78,79;148,149) in a direction such as to disengage the brake member (6;84;166) from the seat (7;94;169) in the absence of a download.

11. A knee prosthesis according to Claim 10, characterised in that control means (5;110) is operably connected to the biasing means to enable the magnitude of the return force provided by the biasing means to be adjusted.

12. A knee prosthesis according to Claim 10 or Claim 11 characterised in that the resilient means comprises a spring (100) recessed in the lower hinge member.

13. A knee prosthesis according to Claim 12 characterized in that the spring (100) is a compression spring having one end located against an abutment (103) in the lower hinge member and having a pull rod (110) extending therethrough, the pull rod having a head (111) at one end engaging the link means (78, 79, 80) and being threaded at its other end to engage a non-rotatably mounted adjustment nut (105) providing an abutment for the other end of the compression spring.

14. A knee prosthesis according to any of the preceding claims characterized in that resilient extensor means (120) is provided to act between the upper and lower hinge members so as to tend to return the knee prosthesis to the unflexed state, the extensor means comprising a stack of spring washers (122).

15. A knee prosthesis according to Claim 14 characterised in that the stack of spring washers (122) is threaded on an extensible stem located on one hinge member and surrounded by a telescoping sleeve (124) located on the other hinge member.

*Fig.1.*

Fig.2.

*Fig.3.*

EP 0 325 347 A1

*Fig.4.*

*Fig.5.*

| | DOCUMENTS CONSIDERED TO BE RELEVANT | | EP 89300116.4 |

| Category | Citation of document with indication, where appropriate, of relevant passages | Relevant to claim | CLASSIFICATION OF THE APPLICATION (Int. Cl.4) |
|---|---|---|---|
| A | DE - B2 - 2 505 322 (KAUFER)<br>* Claims 1,2; fig. 5 *<br><br>-- | 1 | A 61 F 2/38 |
| A | US - A - 4 549 318 (TAKAHAMA)<br>* Claim 1; fig. 2,3,4 *<br><br>-- | 1 | |
| A | US - A - 4 136 405 (PASTRICK)<br>* Claim 1; fig. 1 *<br><br>-- | 1 | |
| A | US - A - 3 990 117 (PRITCHARD)<br>* Claim 1; fig. 7 *<br><br>---- | 1 | |
| | | | **TECHNICAL FIELDS SEARCHED (Int. Cl.4)**<br><br>A 61 F |

| The present search report has been drawn up for all claims | | |
|---|---|---|
| Place of search<br>VIENNA | Date of completion of the search<br>16-03-1989 | Examiner<br>MIHATSEK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503· 03.82